# EUROPEAN PATENT SPECIFICATION

(11) **EP 1 259 241 B1**
(45) Date of publication and mention of the grant of the patent: **05.07.2006**
(21) Application number: 00982786.6
(22) Date of filing: 14.12.2000
(51) Int. Cl.: A61K 31/46, A61P 25/08

(54) **THE USE OF SCOPOLAMINE SALTS**
ANWENDUNG VON SCOPOLAMINSALZEN
UTILISATION DE SELS DE SCOPOLAMINE

(30) Priority: 17.12.1999 BR 9906220
(43) Date of publication of application: 27.11.2002
(73) Proprietor: Fundação de Amparo à Pesquisa do Estado de São Paulo - FAPESP, Alto da Lapa, 05468901 São Paulo, SP (BR)
(72) Inventor: DE MORAES MELLO, Luiz, Eugênio, Aráujo, CEP-05502-060 Sao Paulo, SP (BR); GONCALVES MASSANT, Christina, glesa SAO PAULO, SP (BR); KASTROPIL BENASSI, Simone, Sao Paulo, SP (BR)
(74) Representative: Bongiovanni, Simone
(86) International application number: PCT/BR2000/000137
(87) International publication number: WO 2001/043745

(56) References cited:
- DATABASE WPI Week 199508, Derwent Publications Ltd., London, GB; AN 1995-255471, XP002956012 & CN 1 089 485 A (ZHAO Z) 20 July 1994
- DATABASE WPI Week 199534, Derwent Publications Ltd., London, GB; AN 1995-255461, XP002956013 & CN 1 089 473 A (ZHAO Z) 20 July 1994
- DATABASE CA [Online] LYETH B.G. ET AL.: 'Effects of scopolamine treatment on long-term behavioral deficits following concussive brain unjury to the rat', XP002942614 Database accession no. 109:32080 & BRAIN RES. vol. 452, no. 1-2, 1988, pages 39 - 48

## Description

The current invention relates to a preventive treatment based on the administration of scopolamine as a means to prevent the onset of epilepsy that might ensue after an episode of status epilepticus, head trauma, including those caused by neurosurgical procedures as well as by acute lesional events in general.

Epilepsy is defined as a condition where motor or non-motor seizures recur even in the absence of a toxic-metabolic or febrile background. The above definition clearly distinguishes between a non-epileptic seizure, which is often a single isolated event associated to a metabolic disturbance or intoxication from the true epilepsies. Even though both epileptic and non-epileptic seizures might have a similar clinical manifestation, a convulsive fit, epilepsy implies a permanently altered background condition. In this manner, the nervous system of persons with epilepsy is in some way different in its anatomy, physiology, and pharmacology from that of persons without epilepsy.

Epilepsy has a high prevalence with an annual incidence ranging from 11 to 131/100,000 and a prevalence of 1.5% in the general population (Guerreiro and Guerreiro, 1993). The etiology of this condition, even though unknown in most cases, can sometimes be clearly linked to a previous lesional event. As such, the incidence of epilepsy as a consequence of severe head trauma ranges from 15 to 80% depending on the extent, location and severity of the injured brain area (Gumnit RJ. *Epilepsy and brain injury.* In: *Epilepsy updated: Causes and treatment,* P. Robb (Ed.), Symposia Specialists, Chicago, pp. 177-183, 1980). Indeed the risk for developing epilepsy after severe head trauma is 13 times higher than that of the general population.

Some patients with epilepsy are refractory to the available pharmacological treatment and might be indicated for neurosurgery. However, even in the best medical centers surgical intervention not always results in the complete suppression of seizures (Germano IM, Poulin N and Olivier A. *Reoperation for recurrent temporal lobe epilepsy.* Journal of Neurosurgery, 81: 31-36, 1994). Indeed, it is possible that in those conditions where the epileptic foci has been completely removed and yet there is still the persistence of seizures after surgery, that the surgical act itself might have constituted a brain trauma capable of triggering epilepsy (Sperling MR, Skolnick J and O'Connor MJ. *Prognostic value of auras after Temporal lobectomy.* Epilepsia 38 (Suppl. 8):80, 1997). As already mentioned severe head traumas are clearly associated to the later onset of epilepsy.

The currently available antiepileptic drugs are able to control seizures in 70% of the persons with epilepsy (Yacubian EMT. *Tratamento medicamentoso das epilepsias.* Lemos Editorial, São Paulo, 1999). Despite this relative success in seizure control, the available medication is not really antiepileptic it is rather antiseizure. The available medication for the treatment of epilepsy does not alter or suppress the underlying epileptic condition but merely suppresses the epileptic seizures. Indeed, to date all of the well controlled clinical trials conducted to evaluate a true antiepileptic agent either in patients that already have epilepsy (capable of suppressing epilepsy) either after severe head trauma (capable of preventing epilepsy) have failed so far (Temkin NR. Diknien SS and Winn HR- *Clinical trials for seizure prevention.* In: *Antiepileptic drug development.* J French, I Leppik and MA Dichter (Eds.), Lippincott-Raven Publishers, Philadelphia, pp. 179-88. 1998). Moreover, from CN 1089485, CN 1089473, Lyeth et al. and Rote Liste 1990, it is known the use of scopolamine for preparation of pharmaceutical also in correction with epilepsy. As a consequence, despite the known risk for developing epilepsy the is present after several conditions associated to lesions of the nervous system there is no prophylactic strategy currently available (Mello LEAM. *O desenvolvimento medicamentoso de novas estruturas moleculares.* In: *Tratamento medicamentoso das epilepsias.* EMT Yacubian (Ed.). Lemos Editorial. São Paulo, pp.107-115, 1999; Temkin et al., 1998 *op. Cit.*).

The current invention is aimed at the prophylaxis of the epilepsies that might ensue after severe head trauma. It is based on results with a model of experimental epilepsy in rats. In a first series of experiments in rats it was observed that the selective destruction of a specific neuronal population that synthesizes acetylcholine (basal forebrain cholinergic neurons) suppressed the later onset of epilepsy. Assuming that the results could had been a consequence of the functional blockade of the basal forebrain cholinergic neurons, a reversible pharmacological antagonist of that system was tested. Thus, we used scopolamine, a cholinergic antagonist, to evaluate its potential in preventing the onset of epilepsy in a model of epilepsy in rats.

The current invention was based on an experimental model of epilepsy in which in rats the induction of a brain lesion is accomplished through the administration of pilocarpine, a cholinergic agonist. According to the original description of this model, approximately 20-30 days after pilocarpine injection in either rats or mice, there is the onset of spontaneous recurrent epileptic seizures (Turski WA, Cavalheiro EA, Schwarz M, Czuczwar SJ, Kleinrok Z and Turski L. *Limbic seizures produced by pilocarpine in rats: behavioural, electroencephalographic and neuropathological study. Behavioral Brain Research, 9:315-335,* 1983; Mello LEAM, Cavalheiro EA, Babb TL, Kupfer WR, Pretorius JK, Tan AM and Finch DM. *Circuit mechanisms of seizures in the pilocarpine model of chronic epilepsy: cell loss and mossy fiber sprouting. Epilepsia* 34:985-995, 1993). Due to its special features the pilocarpine model is currently one of the 3 most widely used experimental models in the study of epilepsy.

Until now there has been no experimental procedure that has been shown to be effective in preventing the further development of epilepsy in the pilocarpine or in similar models of epilepsy. In the work developed by the present inventors, it was demonstrated that with the use of escopolamine it is possible to retard or even suppress the onset of spontaneous recurrent seizures, i.e., epilepsy, in both the pilocarpine and the kainate (another agent capable of inducing epilepsy) models of epilepsy. In those animals that eventually went on and developed epileptic seizures, these were at a significantly lesser frequency, as compared to that of animals not treated with scopolamine. The present invention relates accordingly to the use of scopolamine salts as claimed in claim 4.

Scopolamine, together with atropine, is a classic cholinergic agonist and is known to block muscarinic receptors. These drugs are known by their abilities in blocking the cholinergic transmission which is otherwise mediated through muscarinic receptors. For our experiments we used Wistar, adult, male, rats which were maintained under controlled conditions of temperature, light/dark cycles and kept in groups of up to 6 animals in polypropilene boxes with free access to tap water and rat chow pellets. Rats were subject to the intraperitoneal administration of pilocarpine in a dose of 320 mg/Kg for the induction of status epilepticus.

As expected, the systemic administration of pilocarpine lead to a sequence of behavioral alterations such as akinesia, body tremor and/or myoclonic jerks of the head and oro-facial stereotyped movements accompained by profuse salivation. Status epilepticus, a condition of uninterrupt epileptic seizure, developed on average 19 ± 5 min after the injection of pilocarpine. The control group (n=12) did not differ from the experimental group (n=8) with regard to this latency for the onset of status epilepticus. In fact, the animals were randomized and assigned to one of the two groups (control and experimental) based on this latency. Approximately after 90 minutes of the onset of status epilepticus the animals were intraperitoneally injected with sodium thiopental at the dose of 25 mg/Kg, a standard procedure in the pilocarpine model, to diminish the otherwise high mortality rate.

Two hours after the onste of status epilepticus the animals in the experimental group were intraperitoneally injected with the first initial dose of scopolamine (2 mg/kg). This dose is twice that needed to provoke memory impairments in rodents (Rudy JW. *Scopolamine administered before and after training impairs both contextual and auditory fear conditiorting. Neurobiology of Learning and Memory,* 65:73, 1996; Elrod K and Buccafusco JJ. *An evaluation of the mechanisms of scopolamine-induced impairment in two passive avoidance protocols. Pharmacology Biochemistry and Behavior 29:15,* 1998).

For the first 3 consecutive days, scopolamine was given every 6 hours, to allow the maintenance of a steady therapeutic level once its half-life is 2.9 ± 1.2 h. At the end of this three day period (the necessary period to unsure survival to the stress imposed by the induction of status epilepticus), animals were subcutaneously implanted with osmotic pumps (Alzet®, model 2002) to insure a steady therapeutic level of scopolamine for the subsequent 14 days. For the animals in the control group, two hours after the onset of status epileticus, administration of sterile saline solution (0.9% NaCl), rather than scopolamine, was started with a similar protocol.

In the pilocarpine model of epilepsy, a few weeks after the administration of pilocarpine and the induction of status epilepticus, the animals develop spontaneous epileptic seizures which reccur for as long as the animal are allowed to live. It is assumed that such epileptic condition adequately mimics some types of epileptic seizures in humans and may closely reflect the sequence of events that take place in post-traumatic epilepsies.

Approximately 15 to 20 days after the induction of status epilepticus, the reported duration of the latent period for the onset of spontaneous seizures, monitoring of the behavioral seizures with a videocamera for approximately 12 hours/week was initiated. Based on our initial hypothesis, that the experimental group (treated with scopolamine) would either have a substantial reduction or would show no spontaneous epileptic seizures, and therefore to avoid a false negative result, the sampling rate for video monitoring of these animals was performed for 24 hours/week. Animals were video monitored for approximately 120 days after the induction of status epilepticus. The recorded video tapes were routinely assessed in a 29 inches wide television screen and the seizures observed for each animal were recorded.

The latency for the initiation of the video recording sessions was 18.6 ± 2.9 days for the control group and 18.4 ± 2.8 days for the experimental group, and thus within period that has been reported for the spontaneous seizures to initiate. Even though we did not perform video monitoring of the spontaneous seizures from day 1 after the induction of status epilepticus, and despite the sampling basis of our seizure conting method, we believe it is interesting to describe the latency for the onset of spontaneous seizures.

The latency for the onset of spontaneous seizures illustrated in Table 1 clearly demonstrates a difference between the two groups. It is relevant to mention that despite the above mentioned restrictions, regarding the initiation of video monitoring and the sampling nature of such recordings, the encountered values for the control group are strikingly similar to those already reported in the various papers that characterized the epilocarpine model. This similarity therefore help us on validating our procedure for monitoring the spontaneous seizures.

**Table 1. Length of the latent period for the onset of the first recorded spontaneous epileptic seizure for the experimental and the control group**

| | **Latency (days)** |
|---|---|
| CONTROL | 24.9 ± 10.5 |
| | (15-44) |
| EXPERIMENTAL | 54.6 ± 37.5∗ |
| | (18-117) |

| | |
|---|---|
| *Data expressed as mean* ± *standard deviation: number in parenthesis express latency range in days; ∗p< 0.03 (Mann-Whitney).* | |

As it can be seen, the data expressed in Table 1 clearly indicate a protective effect of the adopted therapy (chronic infusion of scopolamine for 14 days) with regard to the onset of spontaneous epiletic seizures in this model. Indeed, the mean latency of the experimental group was twice that of the control group. This effect alone, if replicated in human clinical studies, would already represent a major advance in the control of post-traumatic epilepsies.

The comparison between the control and the experimental groups with regard to seizure frequency did also yield statistically significant differences (see Table 2).

**Table 2. Frequency of spontaneous epileptic seizures for the control and experimental groups.**

| | **Spontaneous seizures/h x 1000** |
|---|---|
| CONTROL (n=12) | 74.49 ± 67.89 |
| | (20-220) |
| EXPERIMENTAL (n=8) | 24.98 ± 28.60* |
| | (0-74) |

| | |
|---|---|
| *Data expressed as mean* ± *standard deviation; number in parenthesis expresss latency range in days; ∗p* < *0.02 (Mann-Whitney).* | |

Table 2 clearly shows that the mean frequency of spontaneous epileptic seizures for the control group was 3 times higher than that for the experimental group (treated with scopolamine). For each individual seizure however, we found no differences between the two groups regarding its qualitative or quantitative nature. The temporal distribution of those seizures over the 120 day period, except for the already mentioned differences, was also similar between the two groups. In both groups, animals having a higher frequency of spontaneous seizures, tended to manifest those seizures in clusters, more than one seizure in one session followed by sessions with no seizures. However, while only 2 animals (25%) of the scopolamine-treated group had seizures in clusters, 7 animals (58%) of the control group showed seizure clustering. Therefore, also in this other feature, the treatment with scopolamine yielded a protection. In the clinical situation, there is no doubt that having seizures in clusters dramatically worsens the quality of life of patients with epilepsy.

One of the most important results was that at the end of the 4 month observation period after the induction of status epilepticus and after 200-300 hours of video monitoring for each animal of the experimental group, there was one animal for which no seizure was recorded and another animal for which only 1 spontaneous seizure was recorded. The control group in comparison, despite a much smaller total number of seizure sampling hours, the animal with the smallest absolute value had 3 recorded spontaneous seizures (corresponding to the value 20 expressed in the lower variation range of the control group in Table 2).

Our results suggest that the chronic administration (e.g., continuous infusion, multiple administrations, transdermic administration) of scopolamine for 14 days after a major head injury might have the potential to diminish or block the later development of epilepsy. In adult, male Wistar rats, the administration of 2 mg/kg every 6 hours followed by the chronic administration of an equivalent dose with osmotic pumps yielded a clear antiepileptogenic effect. The possible use of the current invention in humans will naturally have to rely on clinical trials to establish the appropriate dosages and treatment durations for achieving optimal results. The current results support the development of clinical studies with scopolamine treatment right after the occurrence of major head trauma in humans. Given the frequency of major head trauma and the strong clinical evidence linking this event with the later development of epilepsy it should not be difficult to recruit the necessary number of patients to perform a preliminary assessment of the suggest therapy. Finally it is important to remember that there is currently no available therapy that might be used in this condition.

## Claims

1. The use of scopolamine salts for the preparation of a medicament for the prevention of the development of non-congenital epilepsy.

2. The use of scopolamine salts according to claim 1, **characterized by** the fact that said development of non-congenital epilepsy could be the consequence of accidents with major head trauma, said medicament being aimed to be administered after said accidents with major head trauma to prevent the development of non-congenital epilepsy.

3. The use of scopolamine salts according to claim 1, **characterized by** the fact that said development of non-congenital epilepsy could be the consequence of surgeries for the removal of anatomical structures of epileptogenic nature, said medicament being aimed to be administered after said surgeries for the removal of anatomical structures of epileptogenic nature to prevent the development of non-congenital epilepsy.

## Patentansprüche

1. Verwendung von Scopolaminsalzen für die Herstellung eines Medikaments zur Verhinderung der Entwicklung von nicht-kongenitaler Epilepsie.

2. Verwendung von Scopolaminsalzen nach Anspruch 1, **dadurch gekennzeichnet, dass** diese Entwicklung von nicht-kongenitaler Epilepsie die Folge von Unfällen mit schwerem Hirntrauma sein könnte, wobei dieses Medikament nach diesen Unfällen mit schwerem Hirntrauma verabreicht werden soll, um die Entwicklung von nicht-kongenitaler Epilepsie zu verhindern.

3. Verwendung von Scopolaminsalzen nach Anspruch 1, **dadurch gekennzeichnet, dass** diese Entwicklung von nicht-kongenitaler Epilepsie die Folge von Operationen zur Entfernung von anatomischen Strukturen von epileptogenetischer Natur sein könnte, wobei dieses Medikament nach diesen Operationen zur Entfernung von anatomischen Strukturen von epileptogenetischer Natur verabreicht werden soll, um die Entwicklung von nicht-kongenitaler Epilepsie zu verhindern.

## Revendications

1. Utilisation de sels de scopolamine pour la préparation d'un médicament pour la prévention du développement d'une épilepsie non congénitale.

2. Utilisation de sels de scopolamine selon la revendication 1, **caractérisée par le fait que** ledit développement d'une épilepsie non congénitale pourrait être la conséquence d'accidents avec un traumatisme crânien important, ledit médicament étant destiné à être administré après lesdits accidents avec un traumatisme crânien important pour prévenir le développement d'une épilepsie non congénitale.

3. Utilisation de sels de scopolamine selon la revendication 1, **caractérisée par le fait que** ledit développement d'une épilepsie non congénitale pourrait être la conséquence d'interventions chirurgicales pour l'enlèvement de structures anatomiques de nature épileptogène, ledit médicament étant destiné à être administré après lesdites interventions chirurgicales pour l'enlèvement de structures anatomiques de nature épileptogène pour prévenir le développement d'une épilepsie non congénitale.
